# EUROPÄISCHE PATENTANMELDUNG

(11) **EP 0 759 304 A2**
(43) Veröffentlichungstag der Anmeldung: **26.02.1997**
(21) Anmeldenummer: 96890129.8
(22) Anmeldetag: 22.07.1996
(51) Int. Cl.: A61L 2/12, A61L 2/24

(54) **Anlage zum Erhitzen, Desinfizieren und Steriliseren von Gütern**

(30) Priorität: 24.07.1995 AT 1258/95
(71) Anmelder: Katschnig, Helmut, Dr., A-8750 Judenburg Steiermark (AT)
(72) Erfinder: Katschnig, Helmut Dr., A-8750 Judenburg (AT); Stegmüller, Wolfgang Ing., A-8755 St. Peter/Judenburg (AT); Gruber, Ernst, A-8750 Judenburg (AT)
(74) Vertreter: Itze, Peter, Dipl.-Ing.

(57) **Zusammenfassung**

Die Erfindung betrifft eine Anlage zum Erhitzen, Desinfizieren und Sterilisieren von Gütern, in welcher das zu behandelnde Gut mittels Mikrowellen erhitzt ist, wobei sich das zu behandelnde Gut in einem in die Behandlungskammer (3) eingesetzten Behälter (1) befindet und in den Behälter und damit das zu behandelnde Gut, gegebenenfalls mit Zusätzen versetzte, Flüssigkeit, z.B. Wasser eingebracht wird, wobei für die Flüssigkeitseinbringung eine auf den Behälter aufsetzbare Zugabeeinrichtung vorgesehen ist. Die aufsetzbare Zugabeeinrichtung ist hiebei als dicht auf den Behälterdeckel aufsetzbare Glocke (23) ausgebildet, wobei für die Flüssigkeitseinbringung eine in eine Behälteröffnung (22) einführbare Düse (21) an der Innenseite der Glocke (23) wegragt, und wobei von der Glocke (23) zusätzlich eine Abluft(30)- bzw. Abdampfleitung (28) wegführt.

## Beschreibung

Die Erfindung bezieht sich auf eine Anlage zum Erhitzen, Desinfizieren und Sterilisieren von Gütern, in welchen das zu behandelnde Gut mittels Mikrowellen erhitzt wird, wobei sich das zu behandelnde Gut in einem in die Behandlungskammer eingesetzten Behälter befindet und in den Behälter und damit in das zu behandelnde Gut gegebenenfalls mit Zusätzen versetzte Flüssigkeit, z.B. Wasser, eingebracht wird, wobei für die Flüssigkeitseinbringung eine auf den Behälter aufsetzbare Zugabeeinrichtung vorgesehen ist.

Eine derartige Vorrichtung ist aus der EP-A2 482 104 bekannt. Bei dieser bekannten Ausbildung wird auf eine zentrale Öffnung des Deckels des Behälters eine Zugabeeinrichtung aufgesetzt, welche im wesentlichen lediglich der Wasserzufuhr dient und zusätzlich nur einen Thermofühler und einen Druckfühler enthält. Der im Behälter entstehende Dampf entweicht in die Behandlungskammer und wird von dort abgeführt. Dies hat den Nachteil, daß eine etwa vorhandene Sicherheitseinrichtung zur Ermittlung von Dampf- und/oder Rauchpräsenz relativ spät anspricht, da sich zunächst der Rauch und/oder Dampf in der Behandlungskammer ansammelt und erst dann aus dieser abgeführt wird.

Der Erfindung liegt die Aufgabe zugrunde, eine Anlage der eingangs genannten Art zu schaffen, welche die Überwachungseinrichtungen möglichst zentral angeordnet hat, und welche bei Auftreten von Rauch und/oder Dampf schnell anspricht, um die Leistung der Magnetrons zu steuern.

Erfindungsgemäß wird diese Aufgabe dadurch gelöst, daß die aufsetzbare Zugabeeinrichtung als direkt auf den Behälterdeckel aufsetzbare Glocke ausgebildet ist, wobei für die Flüssigkeitseinbringung eine in eine Behälteröffnung einführbare Düse an der Innenseite der Glocke wegragt und wobei von der Glocke zusätzlich eine Abluft- bzw. Abdampfleitung wegführt. Dadurch wird vermieden, daß im Behälter entstehende Abluft bzw. Abdämpfe in die Behandlungskammer eindringen, wobei die entsprechenden Sicherheitseinrichtungen dann erst viel später ansprechen können.

Vorteilhafterweise kann mit der Abluft- bzw. Abdampfleitung ein Temperaturfühler verbunden sein, wodurch erreicht wird, daß aufgrund der über die Glocke und die Abluft- bzw. Abdampfleitung konzentriert abgeführten Abluft bzw. Abdämpfe der Temperaturfühler sehr schnell anspricht und auch einen realen Wert mißt. Weiters kann in der Abluft- bzw. Abdampfleitung eine Einrichtung zur Erkennung von Rauch und Dampf eingeschaltet sein. Auch in diesem Fall wird durch die konzentrierte Abfuhr der Abluft bzw. des Abdampfes durch die Glocke ein rasches Erkennen des Auftretens von Rauch und/oder Dampf erreicht, was ebenfalls eine Verbesserung der Sicherheit des Betriebs der Anlage wesentlich ist. Dabei kann der Einrichtung zur Erkennung von Rauch eine Kondensationseinrichtung vorgeschaltet sein, wodurch sich aufwendige Differenzierungseinrichtungen zwischen Rauch und Dampf erübrigen. Die Abluftleitung der Kondensationseinrichtung kann dabei in den Abgaskanal der Behandlungskammer insbesondere vor einem Abluftventilator einmünden, wodurch erreicht wird, daß alle Abdämpfe gemeinsam aus der Anlage herausgeführt werden und insbesondere bei Vorsehen eines Abluftventilators vermieden wird, daß sich innerhalb der Behandlungskammer aufgrund von in die Behandlungskammer austretender Dämpfe Kondensat bildet. Die Kondensationseinrichtung kann dabei durch Frischwasser, das zur Einbringung in die Behälter bestimmt ist, gekühlt sein, wodurch eine Vorwärmung des in den Behälter einzubringenden Wassers erreicht wird, was einerseits energiepolitisch und anderseits in zeitlicher Hinsicht sehr günstig ist, da dadurch lange Aufheizzeiten des in den Behälter eingebrachten Wasser entfallen können. Dies führt zu beträchtlichen Verkürzungen der Behandlungszyklen.

Weiters kann mit der Glocke eine Einrichtung zur Erkennung der Absenktiefe verbunden sein. Dies hat den Vorteil, daß über diese Einrichtung ermittelt wird, ob sich tatsächlich ein Behälter innerhalb der Behandlungskammer befindet bzw. kann auch überwacht werden, ob nicht infolge zu hoher Temperatur innerhalb der Behandlungskammer ein Weichwerden des Behälters erfolgt bzw. der Behälter in sich zusammensackt, wodurch dann gleichfalls eine Alarm- und Sicherheitseinrichtung ausgelöst werden kann. Der Temperaturfühler, die Einrichtung zur Erkennung von Rauch und/oder Dampf sowie die Einrichtung zur Erkennung der Absenktiefe der Glocke können mit einer zentralen Steuereinheit verbunden sein, mittels welcher die Energiezufuhr der Mikrowellengeneratoren und/oder die Flüssigkeitszugabeeinrichtung steuerbar sind. Dadurch wird erreicht, daß bei zu hohen Temperaturen oder bei Schmelzen oder Einsacken des Behälters u.dgl. selbsttätig die Anlage ausgeschaltet bzw. wenn im Inneren des Behälters Rauch gemessen wird, direkt über die Flüssigkeitszubringeinrichtung Wasser in den Behälter eingespritzt wird, was den Brand löscht.

Schließlich können mit der Steuereinheit auch ein in der Behandlungskammer befindlicher Thermoschalter und/oder ein ebenfalls in der Behandlungskammer angeordneter Licht- bzw. IR-Sensor verbunden sein, wobei gegebenenfalls in der Behandlungskammer Löschdüsen vorgesehen sind, die gleichfalls über die Steuereinheit geregelt, mit Wasser beaufschlagbar sind. Diese Einrichtung ermöglicht eine zusätzliche Sicherheit dahingehend, daß neben der Überwachung der Vorgänge innerhalb des Behälters auch die Temperatur und eventuell auftretende Brände durch Messung von Temperatur und/oder Licht- bzw. Infrarot erkannt werden können, welche Situationen dann von der zentralen Steuereinheit erkannt werden und durch Einspritzen von Wasser über die zusätzlichen Löschdüsen ein Fortschreiten des Brandes verhindert wird bzw. auch der Behälter soweit wieder abgekühlt wird, daß er formstabil bleibt.

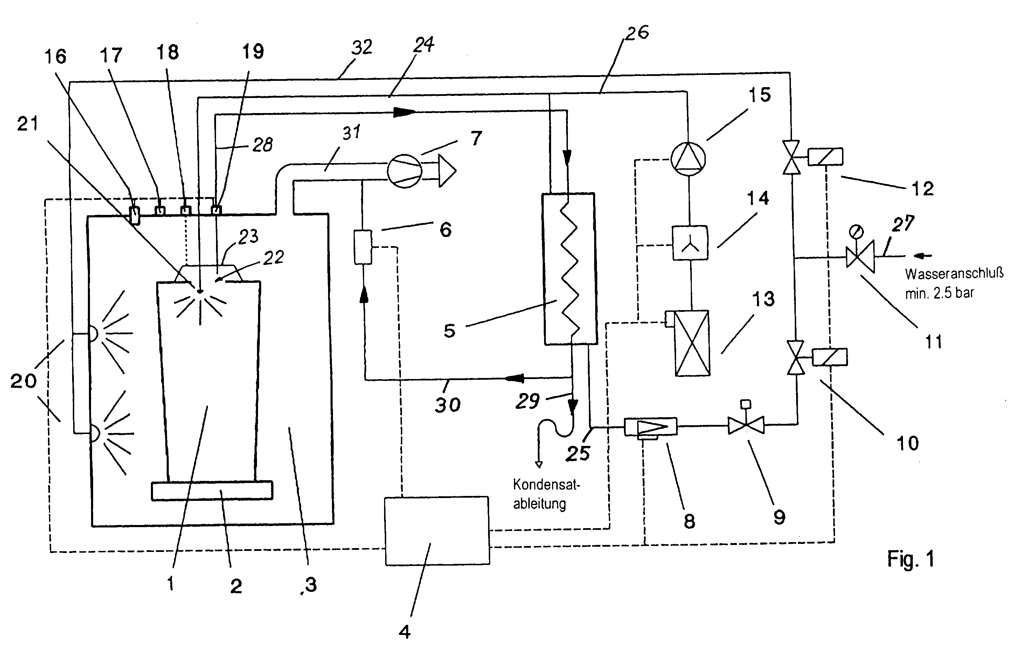

In der Zeichnung (Fig. 1) ist ein Ausführungsbeispiel der erfindungsgemäßen Anlage schematisch wiedergegeben. Mit 1 ist ein Behälter bezeichnet, welcher auf einen Drehteller 2 einer Behandlungskammer 3 einsetzbar ist, welcher in einem Mikrowellengerät vorgesehen ist und welcher in bekannter Weise mittels mehrerer Magnetrone beaufschlagt ist, um tote bzw. kalte Zonen innerhalb der Behandlungskammer zu vermeiden. Der Behälter 1 weist in seinem Deckel eine Öffnung 22 auf, auf welche eine Glocke 23 absenkbar und dicht aufsetzbar ist. Diese Glocke 23 ist in nicht dargestellter Weise an einer Halterung vorgesehen, u.zw. derart, daß sie in der Halterung z.B. über einen Schleifring dicht drehbar gelagert ist. Von dieser Glocke 23 springt eine Düse 21 für die Einbringung von Flüssigkeit vor, und ragt durch die Öffnung 22 in den Behälter 1 hinein, in welchem sich das zu behandelnde Gut befindet. Die Düse 21 ist über eine Leitung 24 mit Flüssigkeit beaufschlagbar, welche von einem Wärmetauscher 5 kommt, in welchen über eine Leitung 25 Frischwasser eingebracht wird. In die Leitung 24 wird über eine Leitung 26, wenn erforderlich, aus einem Behälter 13 Geruchsneutralisator zugeführt, u.zw. mittels einer Pumpe 15, welche die Menge durch einen Durchflußmesser 14 ermittelt. Die in den Wärmetauscher 5 eingebrachte Flüssigkeitsmenge wird über einen Durchflußmesser 8 und ein Nadelventil 9 gesteuert, wobei die grundsätzliche Menge des Wassers über ein Magnetventil 10 regelbar ist. Die Frischwasserleitung, in welcher der Durchflußmesser 8, das Nadelventil 9 und das Magnetventil 10 angeordnet sind, geht von einem zentralen Wasseranschluß 27 aus, über welchen die Flüssigkeit über einen Druckminderer 11 zugeführt wird. Das Magnetventil 10, der Durchflußmesser 8 sowie der Behälter 13 mit Niveauschalter, der Durchflußmesser 14 und die Schlauchpumpe 15 für den Geruchsneutralisator sind mittels Steuerleitungen mit einer zentralen Steuerung 4 verbunden, über welche auch das gesamte System hinsichtlich der Wassermenge und der zugeführten Leistungen der Magnetrons steuerbar ist.

Von der Glocke 23 führt eine Abluft- bzw. Abdampfleitung 28 weg und führt durch den aus Wärmeaustauscher ausgebildeten Kondensator 5 hindurch. Dort wird die Wärme gegen das über die Leitung 25 und 24 der Einspritzdüse 21 zugeführte Frischwasser ausgetauscht, wobei das aus den Dämpfen kondensierende flüssige Medium über die Kondensatleitung 29 abgeführt wird. Die getrocknete gasförmige Phase wird über die Leitung 30 einem Rauchsensor 6 zugeführt, wo ermittelt wird, ob sich in der gasförmigen getrockneten Abluft bereits Rauch befindet. Dieser Rauchsensor ist ebenfalls mit der Steuerung 4 über eine Steuerleitung verbunden. Aus dem Rauchsensor 6 austretende Abluft führt in den Abgaskanal 31, welcher von der Behandlungskammer 3 ausgeht. In diesen Abgaskanal ist ein Abluftventilator 7 eingeschaltet, wobei die getrocknete Abluft in den Abgaskanal 31 vor dem Abluftventilator 7 einmündet.

An der Behandlungskammer 3 ist zusätzlich noch ein Infrarot- oder Lichtsensor 16 vorgesehen, der ebenfalls über eine Steuerleitung mit der Steuerung 4 verbunden ist, desgleichen auch ein Thermoschalter 17 sowie eine Anordnung zur Erkennung der Absenktiefe der Glocke. Auch diese Einleitungen sind über Steuerleitungen mit der Steuerung 4 verbunden.

An der Abluftleitung 28 ist ein Temperatursensor 19 vorgesehen, welcher die Temperatur der aus dem Behälter 1 austretenden Abdämpfe mißt.

An der Behälterkammer sind zusätzlich noch Löschdüsen vorgesehen, welche über eine Leitung 32 und ein Magnetventil 12 mit Wasser beaufschlagbar sind, falls die Steuereinrichtung 4 über den Thermoschalter 17 oder den Infrarot- bzw. Lichtsensor ein Signal gibt, das auf einen Brand innerhalb der Behandlungskammer schließen läßt.

Beim Erfindungsgegenstand handelt es sich somit um ein kombiniertes automatisches System zur Behandlungsgutbefeuchtung und Überwachung für Mikrowellengeräte, wobei bei der Behandlung von Materialien in Mikrowellengeräten, inbesondere von inhomogen zusammengesetzten Abfallmaterialien, die mittels Mikrowellen, z.B. drucklos, thermisch desinfiziert werden sollen, zwei Problempunkte zu lösen sind, nämlich einerseits das gleichmäßige Befeuchten des Gutes und anderseits die Verhinderung von Entzündungsvorgängen aufgrund von zu geringer Befeuchtung. Eine übermäßige Befeuchtung soll anderseits auch vermieden werden, da dies einen unnötigen Energieaufwand bedeuten würde.

Die Befeuchtung von Abfallmaterialien kann entweder manuell durch Wasserzugabe oder automatisch geschehen. Im vorliegenden Fall erfolgt die Befeuchtung vollautomatisch, u.zw. in der gemäß EP-A2 0 483 104 angeführten Weise dadurch, daß der den Behälter 1 aufnehmende Drehteller 2 als Wägeteller ausgebildet ist, wobei aufgrund des ermittelten Gesamtgewichtes gemäß einem bestimmten Algorithmus die erforderliche Wassermenge zugegeben wird. Dabei kann noch auftreten, daß trockene Abfallmaterialien in einem wasserundurchlässigen Abfallsack dicht verschlossen in den Behälter eingeführt werden, sodaß es trotz automatischer Wasserzufuhr geschehen kann, daß die Befeuchtung dieses im wasserundurchlässigen Abfallsack befindlichen Materials nicht erfolgt. Das kann dann, falls der Abfallsack nicht durch Überhitzung eine Öffnung bekommt, durch Erwärmung des Abfallgutes über die Entzündungstemperatur zu Entzündungsvorgängen innerhalb des Behälters führen.

In vorliegender Ausbildung werden daher in erfindungswesentlicher Weise biologische Abfallsäcke verwendet, die sich bei Temperaturen von über 80°C auflösen, wodurch eine generelle gleichmäßige Befeuchtung des gesamten im Behälter 1 befindlichen Abfallgutes während des Behandlungszyklus erreicht wird. 0 Etwa durch Beschickungsfehler auftretende Entzündungsvorgänge werden durch die Infrarotsensoren und den Thermoschalter bzw. auch über den Temperatursensor 19 rechtzeitig erkannt und führen zeitgerecht zu einer Abschaltung der Mikrowellengeneratoren und zum Einbringen von Wasser entweder über die Einspritzdüse 21 oder die Löschdüsen 20.

Beim Betrieb der erfindungsgemäßen Vorrichtung wird der im Behälter befindliche Abfall mit dem Behälter in die Behandlungskammer 3 eingebracht und auf den Drehteller 2 aufgestellt, wobei, wie angeführt, über diesen Drehteller die Menge an zuzuführendem Wasser ermittelt wird. Über eine mechanische Vorrichtung wird durch Schließen der Tür der Behandlungskammer eine elastische Glocke 23 auf den Container aufgesetzt, in der sich die Wassereinspritzdüse 21 und die Leitung 28 zur Ableitung des Abdampfes befinden. Mittels einer Einrichtung 18 zur Erkennung der Absenktiefe der Glocke wird festgestellt, ob sich ein Behälter in der Behandlungskammer befindet, sodaß bei Nichteinsetzen eines Behälters trotz geschlossener Tür die Anlage nicht eingeschaltet werden kann. Die Glocke 23 dichtet gegenüber dem Deckel des Behälters 1 dicht ab, wobei in dem Deckel eine Öffnung 22 vorgesehen ist, durch welche hindurch die Einspritzdüse 21 der Flüssigkeitszuführeinrichtung in den Behälter eingeführt wird. Diese Öffnung 22 könnte durch eine Membran verschlossen sein, wobei dann durch diese Einspritzdüse 21 die Membran beim Aufsetzen der Glocke 23 durchstoßen werden kann. Die Glocke ist an der Absenkeinrichtung drehbar gelagert, d.h., daß die Glocke die Drehbewegung, die der Behälter 1 mittels des Drehtellers 2 erfährt, abfangen kann und die Absenkeinrichtung trotz der Bewegung des Behälters 1 nicht gedreht wird.

Nach Starten des Programmzyklus der Steuerung 4 beginnt sich der Drehteller 2 zu drehen, wobei nach Ermittlung der Flüssigkeitsmenge über das Magnetventil 10, das Nadelventil 9 und den Durchflußmesser 8 Flüssigkeit durch die Einspritzdüse 21 in den Behälter eingespritzt wird, wobei dieser Flüssigkeit einer vorbestimmte Menge an Geruchsneutralisator aus dem Behälter 13 mit Niveauschalter, dem Durchflußmengenzähler und der Schlauchpumpe 15 über die Leitung 26 zugeführt wird.

Nach Einschalten der Mikrowellengeneratoren wird die im Abfall befindliche Flüssigkeitsmenge auf Kochtemperatur gebracht, wobei die Abluft aus dem Container Dampf enthält, dessen Temperatur über einen in der Abdampfleitung vorgesehenen Temperatursensor 19 ermittelt wird. Nach Erreichen der Kochtemperatur schaltet das Gerät automatisch auf die vorgegebene Haltezeit des Desinfektionszyklus um. Die Aufheizzeit, d.h. die Zeit vom Beginn des Einschaltens der Magnetrone bis zum Erreichen der Kochtemperatur im Abfall bzw. zum Erreichen der vorgegebenen Temperatur in der Abluft ist variabel und hängt von der im Behälter 1 befindlichen Flüssigkeitsmenge ab. Dies bedeutet, daß unabhängig von der Abfallzusammensetzung und der Flüssigkeitsmenge im Behälter 1 die vordefinierte Desinfektionszeit erst nach Erreichen der Dampftemperatur im gesamten Abfallgut beginnt. Damit ist ohne Beurteilung des Behandlungsgutes in der erfindungsgemäßen Anlage stets eine maximal sichere Desinfektionswirkung auch bei inhomogenem Abfallgut gewährleistet.

Die aus dem Behälter 1 durch die Glocke 23 über die Leitung 28 austretende Abluft bzw. der Abdampf wird über den Wärmetauscher 5 geführt, durch welchen im Gegenstrom Frischwasser zur Befeuchtung des Behandlungsgutes über die Leitung 24 geführt wird. Über diesen Wärmeaustauscher erfolgt die Aufwärmung des beim nächsten Desinfektionszyklus zugesetzten Wassers, wodurch sich beim zweiten Desinfektionszyklus die Aufheizzeit infolge der Vorwärmung des zugesetzten Wassers deutlich reduziert. Die aus dem Behälter 1 ausgetretene Abluft wird nach dem Wärmetauscher 5 über den Abluftventilator 7 angesaugt, wobei die Abluft den Rauchsensor 6 passiert. Damit können Schwelbrände im Behälter 1 bereits frühzeitig erkannt werden, wodurch sich das Gerät automatisch sofort abschaltet und Wassereinspritzung in den Behälter 1 erfolgt. Der Abluftventilator 7 dient dabei einerseits zur Abfuhr der Abgase aus dem Behälter 1 und anderseits zur Belüftung des Behandlungsraumes 3, um innerhalb des Behandlungsraumes Kondenswasserbildung zu vermeiden.

Kommt es innerhalb des Resonanzraumes zu einer Lichtentwicklung infolge eines Entzündungsvorganges im Abfallgut, so wird dies über den Licht- bzw. Infrarotsensor 16 sofort registriert, welcher dann über die Steuereinrichtung 4 das Gerät abschaltet und gegebenenfalls die Löschdüsen 20 aktiviert. Gleiches kann auch durch den Thermoschalter 17 erfolgen, der als Überhitzungsschalter an der Außenwand der Behandlungskammer 3 ausgebildet ist, wobei bei Ansprechen dieses Überhitzungsschalters ebenfalls die Löschdüsen 20 aktiviert werden und das Gerät abgeschaltet wird.

Über die Einrichtung 18 zur Erkennung der Absenktiefe der Glocke 23, welche mit einem speziellen Höhenschalter verbunden ist, wird erkannt, ob sich ein Behälter 1 in der Behandlungskammer 3 befindet bzw. wenn infolge von Überhitzungen der Behälter schmilzt und sich damit verkleinern sollte. Auch in letzterem Falle schaltet sich das Gerät automatisch ab und es wird sowohl über die Einspritzdüse 21 als auch über die Löschdüsen 20 Flüssigkeit eingespritzt, um so eine weitere Erweichung des Behälters 1 zu verhindern.

Der Behälter 1 wird durch den Drehteller 2 während des gesamten Behandlungszyklus kontinuierlich gedreht, wobei die elastische Glocke, wie schon angeführt, an der Halterung drehbar gelagert ist, u.zw. über einen Schleifring od.dgl., wodurch die Öffnung 22 im Deckel des Behälters 1 hermetisch abgedichtet ist, u.zw. trotz der Bewegung des Behälters 1.

Zusammengefaßt kann festgestellt werden, daß durch die erfindungsgemäße Anlage die Wasser- und Geruchsneutralilsatordosierung optimiert, die Geruchsbelästigung aufgrund des dichten Abschlusses des Behälters 1 vermindert, Energie durch Vorwärmen des zuzuführenden Wassers unter Abkühlung des entstehenden Dampfes, eingespart, die Entzündungsneigung reduziert und die Energieverteilung durch Einbau eines Drehtellers verbessert ist. Dies bedeutet eine weitere Erhöhung der Sicherheit bei der Abfalldesinfektion u.zw. auch bei eventuell auftretenden Fehlbedienungen.

Gegebenenfalls kann die vorliegende Anlage auch unter Über- bzw. Unterdruck betrieben werden.

## Patentansprüche

1. Anlage zum Erhitzen, Desinfizieren und Sterilisieren von Gütern, in welcher das zu behandelnde Gut mittels Mikrowellen erhitzt wird, wobei sich das zu behandelnde Gut in einem in die Behandlungskammer eingesetzten Behälter befindet und in den Behälter und damit das zu behandelnde Gut, gegebenenfalls mit Zusätzen versetzte, Flüssigkeit, z.B. Wasser, eingebracht wird, wobei für die Flüssigkeitseinbringung eine auf den Behälter aufsetzbare Zugabeeinrichtung vorgesehen ist, dadurch gekennzeichnet, daß die aufsetzbare Zugabeeinrichtung als dicht auf den Behälterdeckel aufsetzbare Glocke (23) ausgebildet ist, wobei für die Flüssigkeitseinbringung eine in eine Behälteröffnung (22) einführbare Düse (21) an der Innenseite der Glocke (23) wegragt, und wobei von der Glocke (23) zusätzlich eine Abluft (30)- bzw. Abdampfleitung (28) wegführt.

2. Anlage nach Anspruch 1, dadurch gekennzeichnet, daß mit der Abluft- bzw. Abdampfleitung (28, 30) ein Temperaturfühler (19) verbunden ist.

3. Anlage nach Anspruch 1 oder 2, dadurch gekennzeichnet, daß in der Abluft-bzw. Abdampfleitung (28, 30) eine Einrichtung (6) zur Erkennung von Rauch und/oder Dampf eingeschaltet ist.

4. Anlage nach Anspruch 3, dadurch gekennzeichnet, daß der Einrichtung (6) zur Erkennung von Rauch eine Kondensationseinrichtung (5) vorgeschaltet ist.

5. Anlage nach Anspruch 4, dadurch gekennzeichnet, daß die Abluftleitung (30) der Kondensationseinrichtung (5) in den Abgaskanal (31) der Behandlungskammer (3) insbesondere vor einem Abluftventilator (7) einmündet.

6. Anlage nach Anspruch 4 oder 5, dadurch gekennzeichnet, daß die Kondensationseinrichtung (5) durch Frischwasser, das zur Einbringung in den Behälter (1) bestimmt ist, gekühlt ist.

7. Anlage nach einem der Ansprüche 1 bis 6, dadurch gekennzeichnet, daß mit 30 der Glocke (23) eine Einrichtung (18) zur Erkennung der Absenktiefe verbunden ist.

8. Anlage nach den Ansprüchen 2, 3 und 7, dadurch gekennzeichnet, daß der Temperaturfühler (19), die Einrichtung (6) zur Erkennung von Rauch und/oder Dampf sowie die Einrichtung (18) zur Erkennung der Absenktiefe mit einer zentralen Steuereinheit (4) verbunden sind, mittels welcher die Energiezufuhr der Mikrowellengeneratoren und/oder die Flüssigkeitszugabeeinrichtung steuerbar sind.

9. Anlage nach Anspruch 8, dadurch gekennzeichnet, daß mit der Steuereinheit (4) auch ein in der Behandlungskammer befindlicher Thermoschalter (17) und/oder ein ebenfalls in der Behandlungskammer angeordneter Licht- bzw- IR-Sensor (16) verbunden ist, wobei gegebenenfalls in der Behandlungskammer (3) Löschdüsen (20) vorgesehen sind, die gleichfalls über die Steuereinheit (4) geregelt, mit Wasser beaufschlagbar sind.
